# EUROPÄISCHE PATENTSCHRIFT

(11) **EP 1 572 275 B1**
(45) Veröffentlichungstag und Bekanntmachung des Hinweises auf die Patenterteilung: **24.09.2008**
(21) Anmeldenummer: 03776821.5
(22) Anmeldetag: 29.10.2003
(51) Int. Cl.: A61M 16/00, H03K 17/94

(54) **BEATMUNGSGERÄT, INSBESONDERE CPAP-GERÄT MIT EINER BELEUCHTUNGSEINRICHTUNG**
VENTILATOR, IN PARTICULAR CPAP DEVICE COMPRISING AN ILLUMINATION DEVICE
APPAREIL RESPIRATOIRE, NOTAMMENT APPAREIL CPAP, POURVU D'UN DISPOSITIF D'ECLAIRAGE

(30) Priorität: 19.11.2002 DE 10253934
(43) Veröffentlichungstag der Anmeldung: 14.09.2005
(73) Patentinhaber: Viasys Healthcare GmbH, 97204 Höchberg (DE)
(72) Erfinder: BAECKE, Martin, 06847 Dessau (DE); HEIMHALT, Uwe, 06366 Köthen (DE); ANGER, Ewald, 97246 Eibelstadt (DE); REINSTAEDTLER, Juergen, 97082 Würzburg (DE)
(74) Vertreter: Hellmich, Wolfgang
(86) Internationale Anmeldenummer: PCT/DE2003/003593
(87) Internationale Veröffentlichungsnummer: WO 2004/045692

(56) Entgegenhaltungen:
- WO-A-00/48112
- DE-A- 2 643 912
- US-A- 5 488 273
- US-A- 5 524 101
- US-A- 5 766 155
- US-B1- 6 427 689

## Beschreibung

Die Erfindung bezieht sich auf ein Beatmungsgerät gemäß dem Oberbegriff des Patentanspruchs 1, insbesondere auf ein CPAP-Gerät und eine Steuerung zum Ein- und Ausschalten einer Beleuchtungseinrichtung.

Im Stand der Technik sind "Klatschschalter" bekannt. Diese reagieren auf akustische Impulse, wie z.B. Klatschen. Sie können beispielsweise einen Stecker für das Stromnetz und eine entsprechende Steckdose aufweisen. Über den Stecker und das Stromnetz wird der Klatschschalter mit elektrischer Leistung versorgt. Nach einem ersten Klatschen verbindet er seine Steckdose mit dem Stromnetz, nach dem zweiten Klatschen trennt er die Steckdose wieder vom Stromnetz. Wird der Stecker einer Lampe in die Steckdose des Klatschschalters gesteckt, so kann durch Klatschen Licht ein- und ausgeschaltet werden.

Ferner sind Lampen mit Bewegungsmeldern und Lichtsensoren bekannt. Solche Lampen werden beispielsweise eingesetzt, um Hauseingänge nur dann zu beleuchten, wenn dies erforderlich ist. Meldet der Lichtsensor, dass es dunkel wird, so wird das Licht eingeschaltet, wenn auch der Bewegungssensor die Bewegung einer Person detektiert. Einer ortsunkundigen Person wird insbesondere erspart, in der Dunkelheit nach einem evtl. nicht beleuchteten Schalter zu suchen.

Auch das Display von Mobiltelefonen, wie beispielsweise vom S35 von Siemens, ist mit einer Anzeigebeleuchtung und einer Beleuchtung der Tastenaufschrift ausgerüstet. Nach Betätigen einer beliebigen Taste wird die Anzeigen- und Tastenbeleuchtung eingeschaltet. Wird 10 bis 15 s keine Taste betätigt, so wird die Anzeigen- und Tastenbeleuchtung wieder ausgeschaltet.

Bekannt sind ferner Beatmungsgeräte oder Respiratoren zur maschinellen, künstlichen Beatmung bei allen Formen des Sauerstoffmangelzustands. Sie werden unter anderem für die Langzeitbeatmung eingesetzt. (Roche Lexikon Medizin, 4. Auflage, herausgegeben von der Hoffmann-La Roche AG und Urban & Fischer, Urban & Fischer, München, Stuttgart, Jena, Lübeck, Ulm).

Bekannt sind insbesondere Geräte zur Durchführung der CPAP (continuous positive airway pressure)-Therapie. Die CPAP-Therapie wird in Chest. Volume No. 110, Seiten 1077-1088, Oktober 1996 und Sleep, Volume No. 19, Seiten 184-188 beschrieben. Ein CPAP-Gerät appliziert mittels eines Kompressors, über einen Schlauch und eine Nasenmaske einen positiven Überdruck bis zu etwa 30 mbar in den Atemwegen des Patienten. Dieser Überdruck soll gewährleisten, dass die oberen Atemwege während der gesamten Nacht vollständig geöffnet bleiben und somit keine obstruktiven Atmungsstörungen (Apnoen) auftreten (DE 198 49 571 A1).

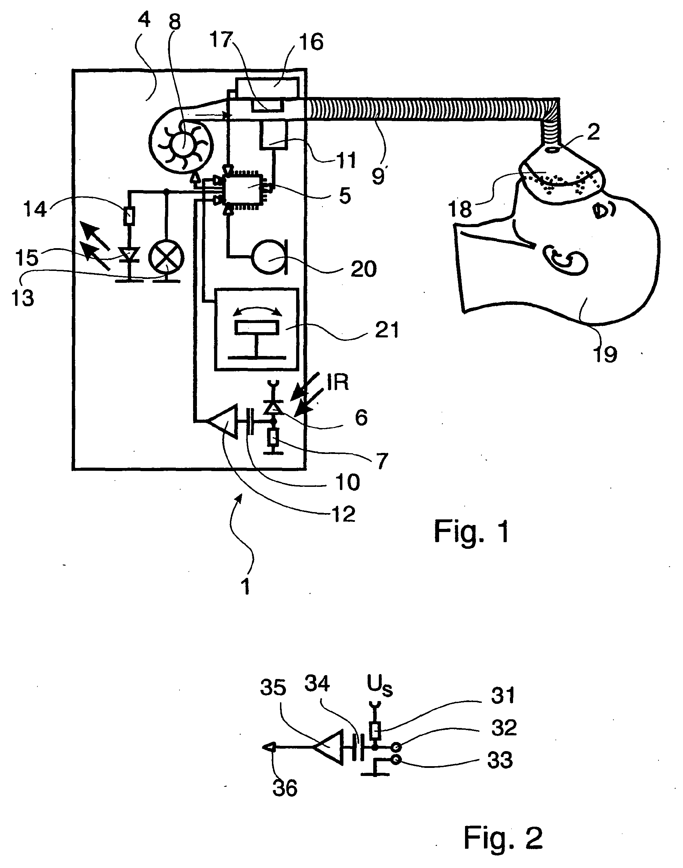

Fig. 1 zeigt CPAP-Gerät 1 und einen Patienten 19. Das CPAP-Gerät wiederum umfasst ein Gehäuse 4, einen Beatmungsschlauch 9 und eine Beatmungsmaske 18. Zur Erzeugung eines Überdrucks enthält das Gehäuse 4 eine Turbine 8. Die Turbine wird auch als Lüfter, Lüftereinheit, Verdichter, Ventilator oder Gebläse bezeichnet. Diese Begriffe werden in diesem Patent synonym verwendet. Im Gehäuse sind ferner ein Drucksensor 11 sowie optional ein Flusssensor 16 untergebracht. In oder nahe bei der Maske sind ein oder mehrere kleine Löcher 2 angebracht, so dass im zeitlichen Mittel ein Luftstrom vom Kompressor zu den Löchern 2 entsteht. Dies verhindert die Anreicherung von CO₂ in Beatmungsschlauch 9 und ermöglicht die Versorgung des Patienten mit Sauerstoff.

Die Drehzahl der Turbine 8 wird durch einen Mikrocontroller 5 so geregelt, dass der mit dem Drucksensor 11 gemessene Istdruck mit einem vorgegebenen Solldruck übereinstimmt. Der Solldruck wird herkömmlicherweise unter Aufsicht eines Arztes voreingestellt und als Titrationsdruck bezeichnet. Der Flusssensor kann z. B. ein Sensor mit Heizdraht 17 sein.

Die WO 00/48112 A1 beschreibt ein medizinisches Gerät, insbesondere eine Infusionspumpe oder ein Beatmungsgerät, mit einer beleuchtbaren Anzeige, die auch ein touch-screen sein kann. Die Anzeige kann von Strahlungssensoren aktiviert werden. Das medizinische Gerät enthält ferner eine Steuerungseinrichtung, die ein Mikroprozessor sein kann.

Die US 5,766,155 beschreibt eine ähnliche Infusionspumpe wie die D1. Zusätzlich ist ein Lichtsensor 18 explizit erwähnt. Fällt das Umgebungslicht unter eine vorbestimmter Schwelle, wird die Anzeigenbeleuchtung 220 aktiviert. Falls keine Taste der Infusionspumpe gedrückt wurde und kein Alarm vorliegt verzweigt ein Programm zu Schritt 714. Die Schritte 714 bis 720 veranlassen, dass die Anzeigenbeleuchtung 220 nach einer vorbestimmten Zeit automatisch ausgeschaltet wird.

Die US 6,427,689 offenbart ein Gerät zur Behandlung von Schlaf-Apnoe, insbesondere ein CPAP-Gerät, das sich über eine Fernbedienung bedienen lässt.

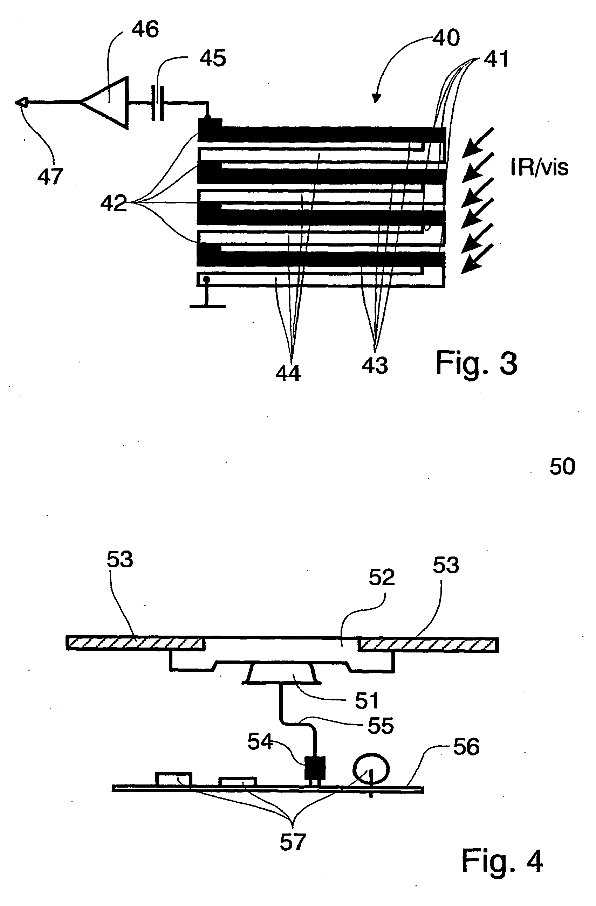

Die US 5,488,273 offenbart einen steuerbaren Deckenventilator mit einer dimmbaren Lichtquelle. Sowohl die Umdrehungsgeschwindigkeit des Deckenventilators als auch die Helligkeit der Lichtquelle können durch Klatschen gesteuert werden. Zu diesem Zweck werden die durch das Klatschen erzeugten Impulswellen durch ein Mikrophon X1 aufgenommen (Fig. 3(a)), durch einen Audioverstärker U2A, U2B verstärkt (Fig. 3(b)) und mit einem Schwellenwert durch einen Operationsverstärker U2C verglichen (Fig. 3(c)). (Spalte 3, Zeilen 9 bis 15:) Falls die zweite oder eine weitere Impulswelle 0,3 bis 0,6 Sekunden nach der ersten beziehungsweise vorangehenden Impulswelle einläuft, wartet das Programm eine weitere Sekunde, um zu prüfen, ob es noch eine weitere Impulswelle gibt. Falls keine weitere Impulswelle empfangen wird, bestimmt das Programm die Art des Steuersignals gemäß der Anzahl der effektiven Impulswellen.

Die US 5,524,101 offenbart eine bewegungsgesteuerte Uhr mit Alarmton und Licht. Gemäß dieser Schrift ist bekannt, pyroelektrische Infrarotdetektoren als Bewegungssensoren einzusetzen. Die Uhrensteuerung ist in Figur 7 und Spalte 4 Zeile 53 bis Spalte 5 Zeile 13 beschrieben. Falls in Schritt 56 mittels Bewegungssensor 16 eine Bewegung ermittelt wird, wird auf jeden Fall die Ziffernblattbeleuchtung eingeschaltet. Falls ferner die Alarmzeit erreicht wurde (Schritt 52), und der Alarm ertönt, wird der Alarm für eine vorbestimmte Schlummerzeit ausgeschaltet.

Es ist die Aufgabe der Erfindung, ein bedienungsfreundliches Beatmungsgerät anzugeben, das die Anzeigenbeleuchtung auch bei durch Tasten hervorgerufenen leichteren Erschütterungen des Beatmungsgeräts aktiviert.

Diese Aufgabe wird durch die Lehre des unabhängigen Anspruchs 1 gelöst.

Bevorzugte Ausführungsformen der Erfindung sind Gegenstand der Unteransprüche.

Vorteilhaft an der Verwerdung eines Mikrofons zur Anschaltung der Beleuchtung ist, dass sich der Patient zur Bedienung des Beatmungsgeräts nicht einmal bewegen muss.

Vorteilhaft am Einschalten der Beleuchtung bei lauten Umgebungsgeräuschen und Ausschalten der Beleuchtung in leiser Umgebung ist, dass dieses Kriterium häufig die Beleuchtung automatisch richtig ein- und ausschaltet. Schläft der Patient, ist es im Schlafzimmer meist leise. Wacht der Patient auf, erzeugt er durch Rascheln mit der Bettdecke Geräusche. Zu diesem Zeitpunkt ist es sinnvoll, die Beleuchtung einzuschalten.

Durch die Detektion von Amplitudenschwankungen der Umgebungsgeräusche kann ermittelt werden, ob in der Umgebung des Beatmungsgeräts gesprochen wird. Der Patient kann das Gerät im Dunklen also ansprechen, um dessen Beleuchtung einzuschalten.

Vorteilhaft an einem Beschleunigungssensor ist, dass er die leichten Erschütterungen registriert, die beispielsweise durch Tasten des Patienten nach dem Beatmungsgerät entstehen.

Ein Infrarotsensor, wie beispielsweise eine Fotodiode oder ein Pyrosensor ist ein preiswerter Bewegungssensor, der auch bei Dunkelheit funktioniert.

Vorteilhaft daran, dass ein Gehäusefenster auf einen Infrarotsensor drückt, ist, dass bei richtiger Wahl des Infrarotsensors auch Bewegungen des Gehäuses und damit des Gehäusefensters zu einem Sensorsignal führen. Auf diese Weise detektiert der Infrarotsensor nicht nur Bewegungen einer Hand vor dem Beatmungsgerät, sondern auch leichte Erschütterungen des Geräts, die durch tastende Bewegungen entstehen.

Vorteilhaft an einer Linse oder Optik vor dem Infrarotsensor ist, dass der Raumwinkel genauer definiert werden kann, indem der Infrarotsensor auf Bewegungen reagiert.

Auch ein Berührungssensor schaltet die Beleuchtung des Beatmungsgeräts vorteilhafterweise ein, wenn der Patient nach dem Beatmungsgerät tastet.

Im Folgenden wird eine bevorzugte Ausführungsform der Erfindung unter Bezugnahme auf die beiliegenden Zeichnungen näher erläutert. Dabei zeigen:
Fig. 1 ein erfindungsgemäßes CPAP-Gerät,
Fig. 2 eine Schaltung für einen Berührungssensor
Fig. 3 Thermosäule zur Infrarotdetektion mit entsprechender Beschaltung und
Fig. 4 den erfindungsgemäßen Einbau eines Infrarotsensors.

Das erfindungsgemäße, in Fig. 1 dargestellte CPAP-Gerät umasst ferner eine Anzeige, vorzugsweise eine LCD (liquid crystal display) und/oder zumindest teilweise durchsichtige Bedienlemente. Anzeige und Bedienelemente können durch eine Beleuchtungseinrichtung beleuchtet werden. Dies können entweder eine oder mehrere Leuchtdioden 15 oder eine oder mehrere Glühbirnen 13 sein. Zum stabilen Betrieb von Leuchtdioden ist noch eine Strombegrenzung z. B. in Form von Widerstand 14 notwendig.

Das CPAP-Gerät umfasst ferner einen Strahlungssensor, welcher als Infrarotsensor (6, 40, 51) ausgebildet ist.

Um Bewegungen in der Nähe des CPAP-Geräts, wie beispielsweise Greifen oder Tasten des Patienten nach dem CPAP-Gerät zu detektieren, wird als preiswerte Lösung der Infrarotsensor eingesetzt. Dies kann entweder eine Fotodiode 6 oder ein Infrarotsensor mit eine Thermosäule 40 sein. Die Fotodiode 6 wird in Sperrrichtung vorgespannt. Je mehr Licht auf die Photodiode fällt, desto höher ist der Strom durch die Fotodiode. Dieser Strom wird beispielsweise an Widerstand 7 in eine Spannung umgewandelt. Da die Fotodiode Bewegungen detektieren soll, wird in vorteilhafter Weise aus der am Widerstand 7 abfallenden Spannung durch den Kondensator 10 deren Wechselanteil der Spannung ausgekoppelt. Das vom Kondensator 10 gelieferte Wechselsignal wird Bauteil 12 zugeführt. Bauteil 12 kann ein bandbegrenzender Verstärker sein oder ein gleichrichtender Verstärker mit anschließendem Tiefpass, um den Mikrocontroller 5 zu entlasten. In einer weiteren Ausführungsform kann Bauteil 12 ein Komparator sein, der bereits ein digitales Ausgangssignal an den Mikrocontroller 5, insbesondere an einen Interrupt-Eingang des Mikrocontrollers 5 liefert.

Darüber hinaus kann das erfindungsgemäße CPAP-Gerät mindestens einen weiteren Sensor enthalten, der beispielsweise ein Mikrofon 20, ein Beschleunigungssensor 21, oder ein Berührungssensor 30 sein kann. Das oder die Sensorsignale werden dem Mikrocontroller 5 zugeführt. Zur Auswertung des Sensorsignals von Drucksensor 11 oder Flusssensor 16 enthält der Mikrocontroller 5 vorzugsweise einen oder mehrere Analog-Digital-Wandler. Handelsübliche Mikrocontroller enthalten oft einen Analog-Digital-Wandler und einen diesem vorgeschalteten Umschalter, so dass beispielsweise einer von acht Analogeingängen mit dem Analog-Digital-Wandler verbunden wird.

Das vom Mikrofon gelieferte Signal kann in einer einfachen Ausführungsform lediglich auf seine Amplitude hin ausgewertet werden. Überschreitet das gleichgerichtete und mit einer Zeitkonstante von 0,3 bis 3 s tiefpassgefilterte Mikrofonsignal einen Schwellenwert, so schaltet der Mikrocontroller die Beleuchtung ein. Das evtl. erforderliche Verstärken des Mikrofonsignals, das Gleichrichten dies Mikrofonsignals und die Tiefpassfliterung kann in einer analogen Schaltung erfolgen oder auch digital im Mikrocontroller 5 durchgeführt werden.

In einer etwas aufwendigeren Ausführungsform können Amplitudenschwankungen des Mikrofonsignals ausgewertet werden. Hierzu wird das Mikrofonsignal ebenfalls gleichgerichtet und anschließend mit einer Zeitkonstante von etwa 0,1 s tiefpassgefiltert. Dieses Amplitudensignal kann wiederum mit einer längeren Zeitkonstante von beispielsweise 10 s tiefpaßgefiltert werden, um ein mittleres Amplitudensignal zu erhalten. Andererseits kann aus dem Amplitudensignal der Gleichanteil beispielsweise mittels eines Kondensators herausgefiltert werden und der Wechselanteil des Amplitudensignals gleichgerichtet und wieder beispielsweise mit einer Zeitkonstante von 1 s tiefpaßgefiltert werden, um ein Amplitudenschwankungssignal zu erhalten. Wenn das Amplitudenschwankungssignal geteilt durch den Amplitudenmittelwert über einem Schwellenwert liegt, schaltet der Mikrocontroller 5 die Beleuchtung ein.

Wird das Beatmungsgerät mit einem leistungsfähigen Mikrocontroller ausgerüstet, so kann der Mikrocontroller eine Spracherkennung durchführen, so dass das Beatmungsgerät komplett durch Sprache steuerbar ist. Die Spracherkennung braucht nicht sehr ausgefeilt sein, da sie nur einige wenige Befehle, wie Druck ein, Druck aus, Beleuchtung ein evtl. Druck erhöhen, Druck absenken usw. verstehen muss.

Durch einen Beschleunigungssensor 21 können die leichten Erschütterungen detektiert werden, die dadurch entstehen, dass der Patient nach dem CPAP-Gerät tastet und dabei leicht auf das CPAP-Gerät schlägt. Um solche leichten Schläge optimal detektieren zu können, wird der Beschleunigungssensor 21 vorzugsweise entfernt von der Standfläche oder Fußnippeln des CPAP-Geräts angeordnet. Steht das CPAP-Gerät auf einem nicht allzu stabilen Regal, so kann der Beschleunigungssensor 21 auch leichte beim Tasten entstehende Erschütterungen des Regalbodens detektieren und anschließend die Beleuchtung einschalten.

Die eine mögliche Beschaltung eines Berührungssensors ist in Fig. 2 dargestellt. Der Widerstand 31 legt die Elektrode 32 auf Spannung Uₛ, wobei in der zuerst besprochenen Ausführungsform die Spannung Uₛ eine Gleichspannung sein soll. Der Kondensator 34 koppelt den Wechselanteil der an Elektrode 32 anliegenden Spannung aus und führt den Wechselanteil dem Bauteil 35 zu. Bauteil 35 kann in der einfachsten Form ein Komparator sein, der beispielsweise eine logische Null entsprechend OV ausgibt, wenn an seinem Eingang eine Spannung unterhalb eines Schwellenwerts liegt und andernfalls eine logische Eins ausgibt, die beispielsweise 5 V entspricht. Berührt nun ein Bediener die Elektroden 32 und 33 gleichzeitig, so stellt der Bediener einen elektrischen Widerstand zwischen diesen beiden Elektroden dar, wodurch die Spannung an Elektrode 32 sinkt. Hierdurch entsteht durch Kondensator 34 ein negativer Spannungsimpuls, der im Bauteil 35 detektiert und in einen digitalen, negativen Impuls umgewandelt werden kann. Insbesondere, wenn Uₛ eine Gleichspannung ist, kann der Kondensator 34 entfallen.

In einer anderen Ausführungsform kann die Spannung Uₛ eine Wechselspannung sein. Durch das gleichzeitige Berühren der Elektroden 32 und 33 nimmt auch im Wechselstromfall die Amplitude der Wechselspannung an Elektrode 32 ab. Dies kann beispielsweise durch einen einfachen Komparator detektiert werden, der ein rechteckförmiges Wechselspannungssignal ausgibt, wenn Elektroden 32 und 33 nicht berührt werden und ein Gleichspannungssignal ausgibt, wenn Elektroden 32 und 33 berührt werden. In einer anderen Ausführungsform kann die vom Kondensator 34 gelieferte Wechselspannung zunächst gleichgerichtet und die Amplitude der gleichgerichteten Wechselspannung mit einem Schwellenwert in Bauteil 35 verglichen werden. Anschluss 36 symbolisiert die Verbindung mit Mikrocontroller 5.

Im Wechselspannungsfall kann die Elektrode 33 wegfallen. Falls ein Patient die Elektrode 32 berührt, wirkt sein Körper als ein großer Kondensator gegen Masse, wodurch auch die Amplitude der Wechselspannung an Elektrode 32 absinkt. Bei dieser Ausführungsform kann sich die Elektrode 32 netzförmig über das Gehäuse des Beatmungsgeräts erstrecken, so dass der Berührungssensor 30 jede Berührung des Gehäuses detektiert. In einer anderen Ausführungsform kann das Gehäuse auch aus einem leitfähigen Kunststoff bestehen, der dann die Elektrode 32 bildet. Die Leitfähigkeit des Kunststoffs braucht nicht hoch sein, da der Widerstand der Haut des Patienten auch im 100 kOhm-Bereich liegt.

Infrarotsensoren, insbesondere für fernes Infrarot, enthalten oft Thermosäulen. Diese messen die Temperaturdifferenz zwischen einer wärmeren Stelle 41 und einer kälteren Stelle 42. Die wärmere Stelle 41 ist so geschwärzt, dass sie in einem möglichst breiten Frequenzbereich Licht absorbiert, also als schwarz erscheint. Die Thermosäule besteht aus einer meanderförmigen Anordnung von zwei unterschiedlichen Leitern, die insbesondere eine unterschiedliche Position in der thermoelektrischen Spannungsreihe aufweisen. Die Thermosäule liefert eine Spannung, die näherungsweise proportional zur einfallenden Lichtleistung ist. Da Bewegungen detektiert werden sollen, wird vorzugsweise über Kondensator 45 der Wechselanteil der Spannung ausgekoppelt, über Verstärker 46 verstärkt und über Anschluss 47 dem Mikrokontroller 5 zugeführt.

Verstärker 46 kann auch durch Bauteil 12 ersetzt werden.

Es hat sich ferner herausgestellt, dass ein Infrarotsensor auch auf leichte Erschütterungen des Gehäuses hin ein Sensorsignal ausgibt, wenn er wie in Fig. 4 eingebaut wird. Fig. 4 zeigt einen Infrarotsensor 51, ein Plexiglasfenster 52, Gehäuseteile 53, Sensorfassung 54, Anschlussdrähte 55, Platine 56 sowie elektrische und elektronische Baueile 57. Wichtig ist, dass der Sensor durch seine Anschlussdrähte mit seinem Fenster gegen das Plexiglasfenster 52 gedrückt wird. Ein Abwinkeln der Anschlussdrähte, wie in Fig. 4 gezeigt, ist hierzu nicht erforderlich. Um jedoch bei der Fertigung auch einen reproduzierbaren mechanischen Kontakt zwischen Infrarotsensor 51 und Plexiglasfenster 52 zu gewährleisten, ist es vorteilhaft, die Anschlussdrähte abzuwinkeln, so dass sie als Federn wirken.

## Patentansprüche

1. Beatmungsgerät insbesondere CPAP-Gerät mit:
einer Beleuchtungseinrichtung (13, 14, 15) zur Beleuchtung einer Anzeige oder von Bedienelementen; und
einer Steuerung (5), die mit der Beleuchtungseinrichtung verbunden ist zum Ein- und Ausschalten der Beleuchtung;
**dadurch gekennzeichnet, dass** das Bestimungsgerät ferner einen Strahlungssensor (6, 40, 51) aufweist, der mit der Steuerung (5) verbunden ist und ein Signal an die Steuerung abgibt,
und, **dass** das Signal die Steuerung veranlasst, die Beleuchtung einzuschalten, wobei der Strahlungssensor ein Infrarotsensor (6, 40, 51) ist, der die Bewegung von Objekten, insbesondere Händen, in der Nähe des Beatmungsgeräts detektiert, wobei der Infrarotsensor (51) so eingebaut ist, dass er gegen einen durchsichtigen Teil (52) des Gehäuses drückt.

2. Beatmungsgerät nach Anspruch 1, wobei die Steuerung die Beleuchtung ausschaltet, wenn die Steuerung eine vorbestimmte Zeit lang kein Signal erhält, das die Steuerung veranlasst, die Beleuchtung einzuschalten.

3. Beatmungsgerät nach einem der obigen Ansprüche, wobei das Beatmungsgerät ferner ein Mikrofon (20) umfaßt.

4. Beatmungsgerät gemäß Anspruch 3, wobei die Steuerung die Höhe des vom Mikrofon gelieferten Signals auswertet und die Beleuchtung einschaltet, wenn die Amplitude den vorgegebenen Schwellenwert überschreitet.

5. Beatmungsgerät nach einem der Ansprüche 3 oder 4, wobei die Steuerung die Amplitude des vom Mikrofon (20) gelieferten Signals auswertet und die Steuerung die Beleuchtung einschaltet, wenn das Verhältnis von Schwankungen der Amplitude zu einem zeitlichen Mittelwert der Amplitude einen Schwellenwert überschreitet.

6. Beatmungsgerät nach einem der obigen Ansprüche, wobei das Beatmungsgerät ferner einen Bewegungssensor (21) mit einem Beschleunigungssensor umfaßt, wobei der Beschleunigungssensor entfernt von der Standfläche des Beatmungsgeräts angebracht ist, um Beschleunigungen des Beatmungsgeräts zu detektieren, die durch Tasten nach dem Beatmungsgerät entstehen, wobei die Steuerung die Beleuchtung einschaltet, wenn das vom Sensor gelieferte Signal Beschleunigungen über einem Schwellenwert anzeigt.

7. Beatmungsgerät gemäß einem der obigen Ansprüche, wobei der Infrarotsensor eine Fotodiode (6) oder ein Pyrosensor (40) ist.

8. Beatmungsgerät nach Anspruch 7, wobei das Beatmungsgerät ferner eine Optik enthält, um Licht aus einem festgelegten Raumwinkelbereich auf den Infrarotsensor (6, 40, 51) zu fokussieren.

## Claims

1. Ventilator, in particular CPAP device, comprising:
an illumination device (13, 14, 15) for illuminating a display (23) or operating members (24); and
a controller (5) connected with the illumination device for switching the illumination on and off;
**characterized in that**
the ventilator further comprises a radiation sensor (6, 40, 51) connected with the controller (5) and outputting a signal to the controller, and that the signal causes the controller to switch on the illumination, wherein the radiation sensor is an infrared sensor (6, 40 51) detecting the movement of objects, in particular of hands, in the proximity of the ventilator, wherein the infrared sensor (51) is installed such that it presses against a transparent part (52) of the housing.

2. Ventilator according to claim 1, wherein the controller switches off the illumination if the controller does not receive a signal for a predetermined time causing the controller to switch on the illumination.

3. Ventilator according to one of the preceding claims, wherein the ventilator further comprises a microphone (20).

4. Ventilator according to claim 3, wherein the controller evaluates the height of the signal supplied by the microphone and switches on the illumination if the amplitude exceeds the predetermined threshold.

5. Ventilator according to one of claims 3 or 4, wherein the controller evaluates the amplitude of the signal supplied by the microphone (20) and the controller switches on the illumination if the ratio of variations of the amplitude divided by a mean-time value of the amplitude exceeds a threshold.

6. Ventilator according to one of the preceding claims, wherein the ventilator further comprises a movement sensor (21) including an accelerometer, the accelerometer being disposed away from the footprint of the ventilator so as to detect accelerations of the ventilator caused by groping for the ventilator, the controller switching on the illumination if the signal supplied by the sensor indicates accelerations above a threshold.

7. Ventilator according to one of the preceding claims, wherein the infrared sensor is a photodiode (6) or a pyrosensor (40).

8. Ventilator according to claim 7, wherein the ventilator further comprises optics (22) so as to focus light from a defined solid angle range onto the infrared sensor (6, 40, 51).

## Revendications

1. Appareil respiratoire, notamment appareil CPAP pourvu:
d'un dispositif d'éclairage (13, 14, 15) pour éclairer un affichage ou des éléments de manoeuvre; et
une commande (5) reliée au dispositif d'éclairage pour connecter et déconnecter l'éclairage;
**caractérisé en ce que**:
l'appareil respiratoire comporte en outre un capteur de rayonnement (6, 40, 51) relié à la commande (5) et envoyant un signal à la commande; et **en ce que**
le signal amène la commande à connecter l'éclairage, le capteur de rayonnement étant un capteur infrarouge (6, 40, 51) qui détecte le déplacement d'objets, notamment de mains, à proximité de l'appareil respiratoire, le capteur infrarouge (51) étant réalisé de telle sorte qu'il appuie contre une partie transparente (52) du boîtier.

2. Appareil respiratoire selon la revendication 1, la commande déconnectant l'éclairage lorsque la commande ne reçoit pendant une certaine période prédéfinie aucun signal amenant la commande à connecter l'éclairage.

3. Appareil respiratoire selon l'une quelconque des revendications précédentes, l'appareil respiratoire comprenant en outre un microphone (20).

4. Appareil respiratoire selon la revendication 3, la commande analysant le niveau du signal fourni par le microphone et connectant l'éclairage lorsque l'amplitude dépasse la valeur seuil prédéfinie.

5. Appareil respiratoire selon l'une quelconque des revendications 3 ou 4, la commande analysant l'amplitude du signal fourni par le microphone (20) et la commande connectant l'éclairage lorsque le rapport des oscillations de l'amplitude par rapport à une valeur moyenne dans le temps de l'amplitude dépasse une valeur seuil.

6. Appareil respiratoire selon l'une quelconque des revendications précédentes, l'appareil respiratoire comprenant en outre un capteur de mouvements (21) avec un capteur d'accélération, le capteur d'accélération étant disposé à distance de la surface d'appui de l'appareil respiratoire, pour détecter les accélérations de l'appareil respiratoire provoquées par des manipulations effectuées à proximité de l'appareil respiratoire, la commande connectant l'éclairage lorsque le signal fourni par le capteur indique la présence d'accélérations au-delà d'une valeur seuil.

7. Appareil respiratoire selon l'une quelconque des revendications précédentes, le capteur infrarouge étant une photodiode (6) ou un capteur pyrométrique (40).

8. Appareil respiratoire selon la revendication 7, l'appareil respiratoire contenant en outre un système optique (22) afin de focaliser la lumière appartenant à une plage angulaire définie sur le capteur infrarouge (6, 40, 51).
